# EUROPÄISCHE PATENTANMELDUNG

(11) **EP 4 702 936 A1**
(43) Veröffentlichungstag der Anmeldung: **04.03.2026**
(21) Anmeldenummer: 25197185.9
(22) Anmeldetag: 21.08.2025
(51) Int. Cl.: A61B 17/34, A61M 5/32, A61M 25/06

(54) **KANÜLE MIT MEHRKANTIGEM KANÜLENROHR UND RETROREFLEKTIERENDER KANÜLENSPITZE**

(30) Priorität: 28.08.2024 DE 102024124599
(71) Anmelder: B. Braun Melsungen AG, 34212 Melsungen (DE)
(72) Erfinder: Weiß, André, 34225 Baunatal (DE); Vukovic, Dejana, 34123 Kassel (DE); Schröder, Tobias, 49205 Hasbergen (DE); Berger, Niclas, 34121 Kassel (DE)
(74) Vertreter: Patentanwälte Ruff, Wilhelm, Beier, Dauster & Partner mbB

(57) **Zusammenfassung**

Die Erfindung betrifft eine Kanüle mit einem längserstreckten Kanülenrohr und einer distalen Kanülenspitze. Erfindungsgemäß weist das Kanülenrohr ein Mehrkanthohlprofil auf und/oder die Kanülenspitze weist eine retroreflektierende Geometrie auf.

## Beschreibung

Die Erfindung betrifft eine Kanüle mit einem längserstreckten Kanülenrohr und einer distalen Kanülenspitze.

Kanülen werden auch als Hohlnadeln bezeichnet und für unterschiedlichste medizinische Anwendungen eingesetzt, in erster Linie zur Punktion von Körpergewebe und anschließender Injektion oder Aspiration von Flüssigkeiten. Zudem können Kanülen beim Anlegen von Kathetern oder sonstigen Invasivkomponenten verwendet werden, wobei das Kanülenrohr in diesem Fall als eine Art Zuführkanal für die betreffende Invasivkomponente dient.

Bei vielen medizinischen Anwendungen ist eine genaue Positionierung der Kanüle im Körpergewebe von besonderer Bedeutung. Kanülen werden deshalb oftmals unter Verwendung bildgebender Ultraschallverfahren positioniert, wobei die Position der Kanüle im Ultraschallbild kontrolliert wird. Dies setzt eine ausreichende Sichtbarkeit der Kanüle im Ultraschallbild voraus. Mit anderen Worten muss die Kanüle muss eine ausreichende Ultraschallsichtbarkeit aufweisen.

Übliche Kanülen weisen ein hohlzylindrisches Profil mit kreisringförmigem Querschnitt auf und sind aus Metall gefertigt, wodurch in der Regel bereits eine ausreichende Ultraschallsichtbarkeit gegeben ist.

Aus dem Stand der Technik sind Kanülen bekannt, die über eine Modifizierung der Oberflächenbeschaffenheit des Kanülenrohrs eine verbesserte Ultraschallsichtbarkeit erzielen sollen.

Aufgabe der Erfindung ist es, eine Kanüle der eingangs genannten Art bereitzustellen, die Vorteile gegenüber dem Stand der Technik bietet. Im Speziellen soll eine verbesserte visuelle Unterscheidbarkeit zwischen dem Kanülenrohr und der Kanülenspitze im Ultraschallbild ermöglicht werden.

Diese Aufgabe wird dadurch gelöst, dass das Kanülenrohr ein Mehrkanthohlprofil aufweist. Alternativ oder zusätzlich weist die Kanülenspitze eine retroreflektierende Geometrie auf. Durch die erfindungsgemäße Lösung weisen das Kanülenrohr und die Kanülenspitze unterschiedliche Reflexionseigenschaften für bei einer Positionskontrolle verwendeten Ultraschallsignale auf. Das Mehrkanthohlprofil des Kanülenrohrs erzeugt ein relativ schwaches Ultraschallecho. Die retroreflektierende Geometrie der Kanülenspitze erzeugt ein relativ starkes Ultraschallecho.

Hierdurch sind die Kanülenspitze und das Kanülenrohr im Ultraschallbild mit unterschiedlicher Deutlichkeit sichtbar, was eine einfache und zuverlässige visuelle Differenzierung zwischen der Kanülenspitze und dem Kanülenrohr im Ultraschallbild und letztlich eine verbesserte Positionskontrolle ermöglicht. Bei einer bevorzugten Ausgestaltung ist das Mehrkanthohlprofil ein Dreikanthohlprofil mit drei Außenlängskanten. Bei weiteren Ausgestaltungen weist das Mehrkanthohlprofil mehr als drei Außenlängskanten auf, beispielsweise vier, fünf oder mehr als fünf Außenlängskanten. Die retroreflektierende Geometrie der Kanülenspitze ist bei unterschiedlichen Ausgestaltungen unterschiedlich ausgeführt. Durch ihre retroreflektierende Geometrie wirkt die Kanülenspitze als Retroreflektor für die Ultraschallsignale des bildgebenden Ultraschallverfahrens. Retroreflektoren sind als solches bekannt und werden auch als Rückstrahler bezeichnet. Unter einem Retroreflektor kann ein Körper oder ein Abschnitt eines Körpers verstanden werden, welcher einfallende Wellen, insbesondere Schallwellen, im Wesentlichen unabhängig von der Einfallsrichtung in Bezug auf eine Ausrichtung des Körpers oder Körperabschnitts überwiegend in diejenige Richtung reflektiert, aus welcher die Wellen emittiert wurden. Diese Wirkung wird auch als Retroreflexion oder Rückstrahlung bezeichnet. Es sind unterschiedliche Ausführungen von Retroreflektoren bekannt, beispielsweise Tripelspiegel, Tripelprismen, linsenähnliche Ausführungen oder dergleichen.

In Ausgestaltung der Erfindung ist das Mehrkanthohlprofil ein Dreikanthohlprofil, das drei längserstreckte Außenlängskanten und drei Außenflächen aufweist, wobei die Außenflächen jeweils zwischen zwei der drei Außenlängskanten erstreckt sind. Die Gestaltung als Dreikanthohlprofil erlaubt eine maximale Reduktion des Ultraschallechos des Kanülenrohrs. Der Kontrast zwischen dem Kanülenrohr und der Kanülenspitze im Ultraschallbild ist hierdurch besonders stark. Die Kanülenspitze kann deshalb besonders sicher identifiziert werden. Durch die Gestaltung als Dreikanthohlprofil wird praktisch kein Ultraschallecho an die Ultraschallquelle des bildgebenden Ultraschallverfahrens zurückgeworfen, da in der Regel keine der drei Außenflächen senkrecht zu einer Schallebene des Ultraschallkopfs des bildgebenden Ultraschallverfahrens ausgerichtet ist. Vorzugsweise sind die drei Außenflächen jeweils eben. Vorzugsweise sind die drei Außenlängskanten jeweils gerade längserstreckt.

In weiterer Ausgestaltung der Erfindung weist das Dreikanthohlprofil drei längserstreckte Innenlängskanten und drei Innenflächen auf, wobei die drei Innenflächen jeweils zwischen zwei der drei Innenlängskanten erstreckt sind. Bei dieser Ausgestaltung der Erfindung ist folglich nicht nur ein Außenumfang des Kanülenrohrs dreikantig oder dreieckig ausgeführt, sondern auch der Innenumfang. Es ist aber auch denkbar und möglich, dass das Dreikanthohlprofil eine kreisrunde oder auf sonstige Weise gestaltete Querschnittsöffnung aufweist.

In weiterer Ausgestaltung der Erfindung ist das Dreikanthohlprofil gleichschenklig, wodurch die Außenflächen an den Außenlängskanten und/oder die Innenflächen an den Innenlängskanten jeweils einen Winkel von 60° einschließen. Durch die gleichschenklige Gestaltung des Dreikanthohlprofils ergibt sich ein in Bezug auf unterschiedliche Ausrichtungen des Kanülenrohrs möglichst unveränderlich geringes Ultraschallecho. Überdies wird die Fertigung der Kanüle vereinfacht. Hierdurch können Kosten eingespart werden.

In weiterer Ausgestaltung der Erfindung ist das Mehrkanthohlprofil an seinen Außenlängskanten mit jeweils einem Außenradius abgerundet. Diese Ausgestaltung der Erfindung geht von der Überlegung aus, dass nicht abgerundete Außenlängskanten bei einer Drehung der Kanüle im Körpergewebe ein potenzielles Verletzungsrisiko darstellen. Durch die Abrundung der Außenlängskanten mit jeweils einem Außenradius wird solchen Verletzungen vorgebeugt. Das Ultraschallecho des Mehrkanthohlprofils wird durch die Außenradien nicht oder allenfalls in praktisch unerheblichem Umfang verstärkt.

In weiterer Ausgestaltung der Erfindung ist die retroreflektierende Geometrie der Kanülenspitze durch einen Materialabtrag, insbesondere Anschliff, des Mehrkanthohlprofils gebildet. Zum Ausbilden der retroreflektierenden Geometrie kann ein distales Ende des Kanülenrohrs schräg abgetragen, insbesondere angeschliffen, sein. In diesem Fall bildet der schräge Materialabtrag und/oder Anschliff des Mehrkanthohlprofils die retroreflektierende Geometrie der Kanülenspitze aus.

In weiterer Ausgestaltung der Erfindung weist die retroreflektierende Geometrie wenigstens eine erste Fläche, eine zweite Fläche und eine dritte Fläche auf, wobei die erste Fläche rechtwinklig zu der zweiten Fläche und rechtwinklig zu der dritten Fläche orientiert ist, und wobei die zweite Fläche und die dritte Fläche aufeinander zugewandt sind. Durch die erste Fläche, zweite Fläche und dritte Fläche und deren Orientierungen zueinander kann das Ultraschallecho der Kanülenspitze weiter maximiert werden. Vorzugsweise liegen die zweite Fläche und die dritte Fläche einander direkt gegenüber. Mit anderen Worten: Vorzugsweise liegen die zweite Fläche und die dritte Fläche in Bezug auf die erste Fläche auf einer gemeinsamen Höhe. Vorzugsweise sind die erste Fläche und die zweite Fläche und die dritte Fläche jeweils eben.

In weiterer Ausgestaltung der Erfindung ist die erste Fläche durch einen Flächenabschnitt einer Innenfläche des Mehrkanthohlprofils gebildet, und die zweite Fläche und die dritte Fläche sind jeweils durch einen Flächenabschnitt einer Wandungsfläche gebildet, wobei die Wandungsflächen jeweils zwischen einer Innenfläche und einer Außenfläche des Mehrkanthohlprofils erstreckt sind. Die zweite Fläche und die dritte Fläche werden bei dieser Ausgestaltung der Erfindung vorzugsweise durch einen Materialabtrag und/oder Anschliff des distalen Kanülenrohrs freigelegt.

Weitere Vorteile und Merkmale der Erfindung ergeben sich aus den Ansprüchen sowie aus der nachfolgenden Beschreibung eines bevorzugten Ausführungsbeispiels der Erfindung, das anhand der Zeichnungen dargestellt ist.

Es zeigen:
- Fig. 1: in schematisch vereinfachter, proximal abgeschnittener Seitenansicht eine Ausführungsform einer erfindungsgemäßen Kanüle mit einem Kanülenrohr und einer distalen Kanülenspitze,
- Fig. 2: das Kanülenrohr der Kanüle nach Fig. 1 in einem Querschnitt entlang einer Schnittlinie II-II gemäß Fig. 1,
- Fig. 3: die Kanülenspitze der Kanüle nach Fig. 1 in einem Querschnitt entlang einer Schnittlinie III-III gemäß Fig. 1,
- Fig. 4: eine schematisch vereinfacht dargestellte exemplarische Verwendungssituation der Kanüle nach Fig. 1 zur Verdeutlichung der reduzierten Ultraschallsichtbarkeit des Kanülenrohrs,
- Fig. 5: in einer der Fig. 4 entsprechenden Darstellungsweise ein aus dem Stand der Technik bekanntes Kanülenrohr mit einem kreisringförmigen Hohlprofil, das ein vergleichsweise starkes Ultraschallecho aufweist,
- Fig. 6: eine schematische Darstellung einer weiteren exemplarischen Verwendungssituation der Kanüle nach Fig. 1 zur Verdeutlichung der retroreflektierenden Eigenschaften der Kanülenspitze, wobei die Kanüle eine erste Orientierung in Bezug auf eine bildgebende Ultraschallquelle einnimmt,
- Fig. 7: eine schematische Darstellung einer weiteren exemplarischen Verwendungssituation entsprechend Fig. 6, wobei die Kanüle eine zweite Orientierung in Bezug auf die Ultraschallquelle einnimmt, und
- Fig. 8: in vereinfachter und abgeschnittener Perspektivansicht die Kanüle nach Fig. 1 im Bereich der distalen Kanülenspitze.

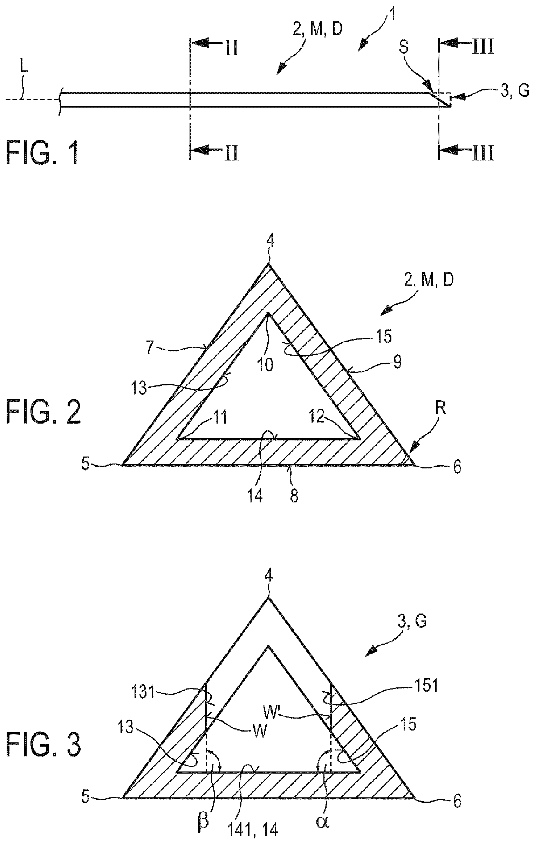

Gemäß Fig. 1 weist eine Kanüle 1 ein längserstrecktes Kanülenrohr 2 und eine distale Kanülenspitze 3 auf.

Die Kanüle 1 kann auch als Hohlnadel bezeichnet werden und dient der Punktion von Körpergewebe zwecks anschließender Injektion und/oder Aspiration von Flüssigkeit. Alternativ oder zusätzlich kann die Kanüle 1 zum Einführen von Invasivkomponenten, wie beispielsweise Führungsdrähten oder Kathetern, in den Körper eines Patienten verwendet werden.

Das Kanülenrohr 2 ist entlang einer Längsachse L gerade längserstreckt. In Fig. 1 ist das Kanülenrohr 2 proximal abgeschnitten dargestellt. Die Kanülenspitze 3 ist an einem distalen Ende des Kanülenrohrs 2 angeordnet. Mit anderen Worten: Die Kanülenspitze 3 bildet das distale Ende des Kanülenrohrs 2.

Das Kanülenrohr 2 weist ein Mehrkanthohlprofil M auf, dessen Querschnitt im Detail in Fig. 2 gezeigt ist. Die Kanülenspitze 3 weist eine retroreflektierende Geometrie G auf, deren Querschnitt im Detail in Fig. 3 gezeigt ist.

Das Mehrkanthohlprofil M weist im Vergleich zu der retroreflektierenden Geometrie G ein schwaches Ultraschallecho auf. Umgekehrt weist die retroreflektierende Geometrie G im Vergleich zu dem Mehrkanthohlprofil M ein starkes Ultraschallecho auf. Wird die Kanüle 1 unter Verwendung eines bildgebenden Ultraschallverfahrens im Körpergewebe positioniert, kann das ausführende medizinische Personal in dem sich ergebenden Ultraschallbild einfach und zuverlässig zwischen dem Kanülenrohr 2 und der Kanülenspitze 3 differenzieren. Dies erlaubt eine verbesserte Positionierung der Kanüle 1.

Bei der gezeigten Ausführungsform ist das Mehrkanthohlprofil M ein Dreikanthohlprofil D (siehe Fig. 2). Das Dreikanthohlprofil D weist drei längserstreckte Außenlängskanten 4, 5, 6 und drei Außenflächen 7, 8, 9 auf. Die Außenlängskanten 4, 5, 6 können auch als erste Außenlängskante 4, zweite Außenlängskante 5 und dritte Außenlängskante 6 bezeichnet werden. Die Außenflächen 7, 8, 9 können auch als erste Außenfläche 7, zweite Außenfläche 8 und dritte Außenfläche 9 bezeichnet werden.

Die Außenflächen 7, 8, 9 sind jeweils eben. Bei einer in den Figuren nicht gezeigten Ausführungsform sind die Außenflächen jeweils konkav eingewölbt. Alternativ können die Außenflächen konvex ausgewölbt sein.

Die erste Außenfläche 7 ist zwischen der ersten Außenlängskante 4 und der zweiten Außenlängskante 5 erstreckt. Die zweite Außenfläche 8 ist zwischen der zweiten Außenlängskante 5 und der dritten Außenlängskante 6 erstreckt. Die dritte Außenfläche 9 ist zwischen der dritten Außenlängskante 6 und der ersten Außenlängskante 1 erstreckt.

Bei der gezeigten Ausführungsform erstreckten die Außenlängskanten 7, 8, 9 sich entlang der Längsachse L jeweils über eine gesamte Länge des Kanülenrohrs 2.

Bei der gezeigten Ausführungsform weist das Dreikanthohlprofil D drei längserstreckte Innenlängskanten 10, 11, 12 und drei Innenflächen 13, 14, 15 auf. Mit anderen Worten: Sowohl ein Außenumfang als auch ein Innenumfang des Kanülenrohrs 2 sind dreieckig oder auch dreikantig gestaltet.

Bei einer in den Figuren nicht gezeigten Ausführungsform ist der Innenumfang abweichend von dem Außenumfang rund, insbesondere kreisrund oder oval, ausgeführt. Bei einer weiteren Ausgestaltung weist der Innenumfang eine mehrkantige Gestaltung mit mehr als drei Innenlängskanten auf, beispielsweise vier, fünf, sechs oder mehr als sechs Innenlängskanten.

Die Innenlängskanten 10, 11, 12 können auch als erste Innenlängskante 10, zweite Innenlängskante 11 und dritte Innenlängskante 12 bezeichnet werden. Die Innenflächen 13, 14, 15 können auch als erste Innenfläche 13, zweite Innenfläche 14 und dritte Innenfläche 15 bezeichnet werden.

Die erste Innenfläche 13 ist zwischen der ersten Innenlängskante 10 und der zweiten Innenlängskante 11 erstreckt. Die zweite Innenfläche 14 ist zwischen der zweiten Innenlängskante 11 und der dritten Innenlängskante 12 erstreckt. Die dritte Innenfläche 15 ist zwischen der dritten Innenlängskante 12 und der ersten Innenlängskante 10 erstreckt.

Die Innenflächen 13, 14, 15 sind jeweils eben. Die Innenflächen 13, 14, 15 sind vorliegend über eine gesamte Länge des Kanülenrohrs 2 entlang der Längsachse L längserstreckt.

Die erste Außenfläche 7 und die erste Innenfläche 13 sind zueinander parallel. Entsprechendes gilt mutatis mutandis für die zweite Außenfläche 8 und die zweite Innenfläche 14 sowie für die dritte Außenfläche 9 und die dritte Innenfläche 15.

Bei der gezeigten Ausführungsform ist das Dreikanthohlprofil D gleichschenklig. Demnach sind die Außenflächen 7, 8, 9 an den Außenlängskanten 4, 5, 6 und die Innenflächen 13, 14, 15 an den den Innenlängskanten 10, 11, 12 jeweils in einem Winkel von 60° zueinander orientiert.

Bei der gezeigten Ausführungsform ist das Dreikanthohlprofil D an seinen Außenlängskanten 4, 5, 6 mit jeweils einem Außenradius R abgerundet. Der Außenradius R ist in Fig. 2 lediglich in Bezug auf die dritte Außenlängskante 6 zeichnerisch angedeutet.

Die retroreflektierende Geometrie G ist bei der gezeigten Ausführungsform durch einen Materialabtrag S des Mehrkanthohlprofils M gebildet. Bei dem Materialabtrag S handelt es sich vorliegend um einen Anschliff. Die Kanülenspitze 3 wird folglich durch ein schräges Abschleifen des distalen Endes des Kanülenrohrs 2 gebildet.

Bei der gezeigten Ausführungsform weist die retroreflektierende Geometrie G wenigstens eine erste Fläche 141, eine zweite Fläche 131 und eine dritte Fläche 151 auf (siehe Fig. 3). Die erste Fläche 141 ist rechtwinklig zu der zweiten Fläche 131 und rechtwinklig zu der dritten Fläche 151 orientiert. Die in Fig. 3 eingezeichneten Winkel α, β entsprechen demnach jeweils 90°. Die zweite Fläche 131 und die dritte Fläche 151 sind aufeinander zugewandt. Dabei liegen die zweite Fläche 131 und die dritte Fläche 151 einander unmittelbar gegenüber. In Bezug auf die erste Fläche 141 liegen die zweite Fläche 131 und die dritte Fläche 151 auf einer gemeinsamen Höhe.

Bei der gezeigten Ausführungsform sind die erste Fläche 141, die zweite Fläche 131 und die dritte Fläche 151 jeweils eben.

Bei der gezeigten Ausführungsform ist die erste Fläche 141 durch einen Flächenabschnitt der dritten Innenfläche 14 gebildet. Die zweite Fläche 131 und die dritte Fläche 151 sind jeweils durch einen Flächenabschnitt einer Wandungsfläche W, W' gebildet, die zwischen Innenflächen und Außenflächen des Mehrkanthohlprofils M erstreckt sind. Dabei ist die zweite Fläche 131 zwischen der ersten Innenfläche 13 und der ersten Außenfläche 7 erstreckt. Die dritte Fläche 151 ist zwischen der dritten Innenfläche 15 und der dritten Außenfläche 9 erstreckt. Die Wandungsflächen W, W und damit die zweite Fläche 131 und die dritte Fläche 151 werden durch den besagten Materialabtrag S freigelegt.

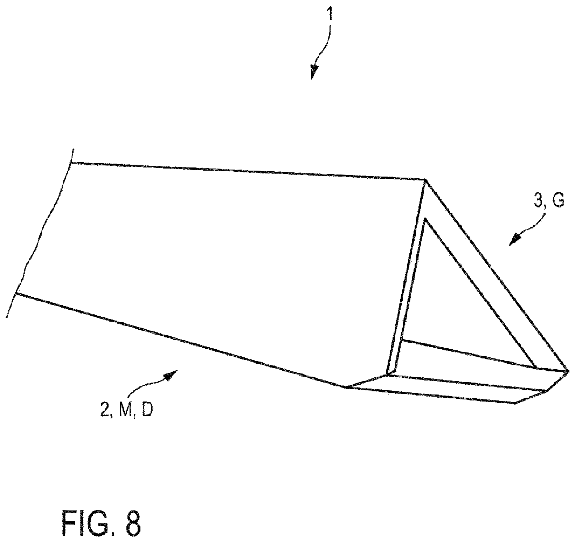

Die in den Fig. 3, 6, 7 sowie 8 gezeigte Gestaltung der retroreflektierenden Geometrie G ist als schematisch aufzufassen. Bei in den Figuren nicht gezeigten Ausführungsformen weist die retroreflektierende Geometrie eine unterschiedliche Gestaltung auf, wobei insbesondere mehr oder weniger Flächen sowie unterschiedliche Orientierungen der Flächen vorgesehen sein können. Mit anderen Worten: Die Kanülenspitze 3 kann auf unterschiedliche Weise als Retroreflektor gestaltet sein. Die in den Figuren gezeigte Gestaltung ist als rein exemplarisch zu verstehen.

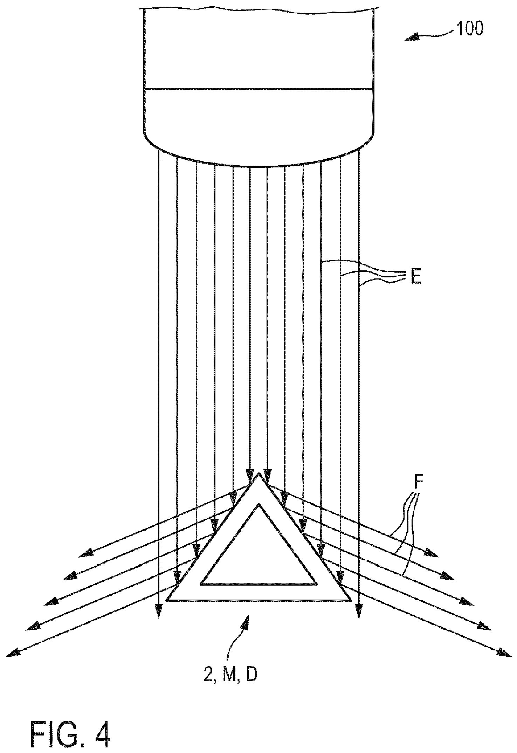

In Fig. 4 ist das Kanülenrohr 2 im Querschnitt zusammen mit einem Ultraschallkopf 100 eines bildgebenden Ultraschallsystems in einer exemplarischen Verwendungssituation gezeigt. Der Ultraschallkopf 100 sendet Ultraschallsignale, die an dem Kanülenrohr 2 reflektiert werden. Die gesendeten Ultraschallsignale sind in Fig. 4 mit dem Bezugszeichen E belegt. Die reflektierten Ultraschallsignale sind mit dem Bezugszeichen F belegt. Durch die Gestaltung des Kanülenrohrs 2 als Mehrkanthohlprofil M, im Speziellen als Dreikanthohlprofil D, werden die gesendeten Ultraschallsignale E in der gezeigten Verwendungssituation von dem Ultraschallkopf 100 wegweisend reflektiert. Das Kanülenrohr 2 hat aufgrund seiner spezifischen Gestaltung kein mittels des Ultraschallkopfs 100 ermittelbares Ultraschallecho. Das Kanülenrohr 2 weist folglich keine oder eine lediglich sehr geringe Ultraschallsichtbarkeit auf.

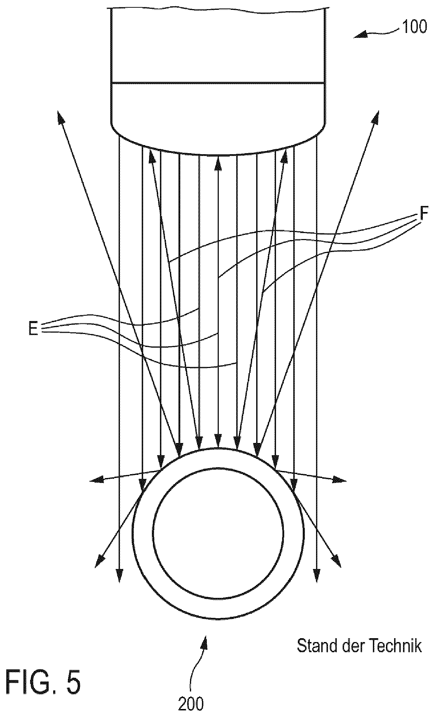

Hierzu im Unterschied ist in Fig. 5 ein aus dem Stand der Technik bekanntes Kanülenrohr 200 mit kreisringförmigem Querschnitt gezeigt. Aufgrund der kreisringförmigen Gestaltung des Kanülenrohrs 200 werden die gesendeten Ultraschallsignale E jedenfalls anteilig zurück in Richtung des Ultraschallkopfs 100 reflektiert. Die reflektierten Ultraschallsignale F können mittels des Ultraschallkopfs 100 empfangen werden. Das aus dem Stand der Technik bekannte Kanülenrohr 200 weist deshalb ein stärkeres Ultraschallecho und damit eine deutlichere Ultraschallsichtbarkeit als das Kanülenrohr 2 nach den Fig. 1 bis 4 auf.

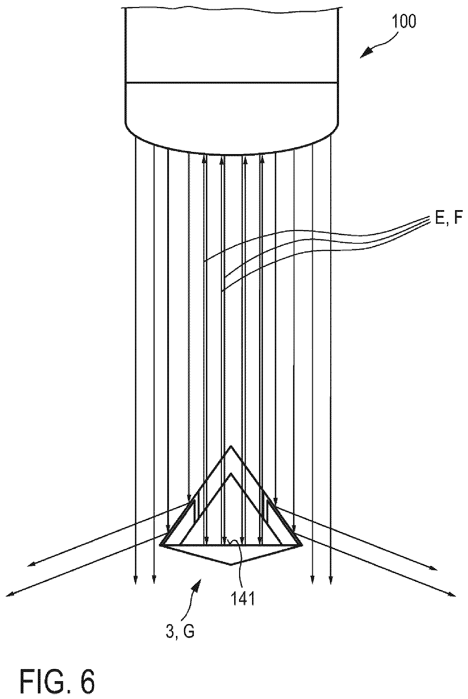

In Fig. 6 ist die Kanülenspitze 3 im Querschnitt zusammen mit dem Ultraschallkopf 100 gezeigt. Die retroreflektierende Geometrie G der Kanülenspitze 3 bewirkt, dass die gesendeten Ultraschallsignale E möglichst vollständig in Richtung des Ultraschallkopfs 100 zurückreflektiert werden. Die reflektierten Ultraschallsignale F bewirken ein vergleichsweise starkes Ultraschallecho und damit eine deutliche Ultraschallsichtbarkeit der Kanülenspitze 3. In der in Fig. 6 gezeigten Verwendungssituation ist die erste Fläche 141 der retroreflektierenden Geometrie G unmittelbar unterhalb und in etwa parallel zu dem Ultraschallkopf 100 ausgerichtet. Die reflektierten Ultraschallsignale F werden hauptsächlich über die erste Fläche 141 in Richtung des Ultraschallkopfs 100 zurückreflektiert.

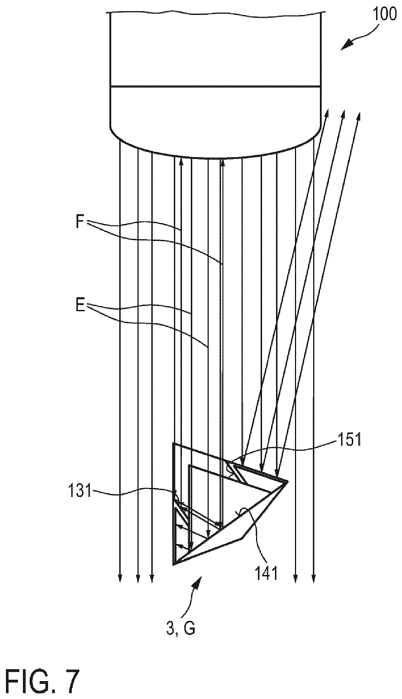

In Fig. 7 ist die Kanüle 1 relativ zu der in Fig. 6 gezeigten Position entgegen dem Uhrzeigersinn um ihre Längsachse L gedreht. Hierdurch ergibt sich eine unterschiedliche Orientierung der retroreflektierenden Geometrie G und ihrer Flächen 141, 131, 151 in Bezug auf den Ultraschallkopf 100. Weitestgehend unabhängig von dieser unterschiedlichen Drehposition ergibt sich dennoch ein starkes Ultraschallecho der Kanülenspitze 3. Dies aufgrund der Gestaltung als Retroreflektor. In der gezeigten Verwendungssituation werden Ultraschallsignale E von der ersten Fläche 141 in Richtung der zweiten Fläche 131 und von dort zurück zu dem Ultraschallkopf 100 reflektiert.

## Patentansprüche

1. Kanüle (1) mit einem längserstreckten Kanülenrohr (2) und einer distalen Kanülenspitze (3), **dadurch gekennzeichnet, dass** das Kanülenrohr (2) ein Mehrkanthohlprofil (M) aufweist, und/oder dass die Kanülenspitze (3) eine retroreflektierende Geometrie (G) aufweist.

2. Kanüle (1) nach Anspruch 1, wobei das Mehrkanthohlprofil (M) ein Dreikanthohlprofil (D) ist, das drei längserstreckte Außenlängskanten (4, 5, 6) und drei Außenflächen (7, 8, 9) aufweist, die zwischen jeweils zwei der drei Außenlängskanten (4, 5, 6) erstreckt sind.

3. Kanüle (1) nach Anspruch 2, wobei das Dreikanthohlprofil (D) drei längserstreckte Innenlängskanten (10, 11, 12) und drei Innenflächen (13, 14, 15) aufweist, die jeweils zwischen zwei der drei Innenlängskanten (10, 11, 12) erstreckt sind.

4. Kanüle (1) nach Anspruch 2 oder 3, wobei das Dreikanthohlprofil (D) gleichschenklig ist, wodurch die Außenflächen (7, 8, 9) an den Außenlängskanten (4, 5, 6) und/oder die Innenflächen (13, 14, 15) an den Innenlängskanten (10, 11, 12) jeweils einen Winkel von 60° einschließen.

5. Kanüle (1) nach einem der vorhergehenden Ansprüche, wobei das Mehrkanthohlprofil (M) an seinen Außenlängskanten (4, 5, 6) jeweils mit einem Außenradius (R) abgerundet ist.

6. Kanüle (1) nach einem der vorhergehenden Ansprüche, wobei die retroreflektierende Geometrie (G) der Kanülenspitze (3) durch einen Materialabtrag (S), insbesondere einen Anschliff, des Mehrkanthohlprofils (M) gebildet ist.

7. Kanüle (1) nach einem der vorhergehenden Ansprüche, wobei die retroreflektierende Geometrie (G) wenigstens eine erste Fläche (141), eine zweite Fläche (131) und eine dritte Fläche (151) aufweist, wobei die erste Fläche (141) rechtwinklig zu der zweiten Fläche (131) und rechtwinklig zu der dritten Fläche (151) orientiert ist, und wobei die zweite Fläche (131) und die dritte Fläche (151) aufeinander zugewandt sind.

8. Kanüle (1) nach Anspruch 7, wobei die erste Fläche (141) durch einen Flächenabschnitt einer Innenfläche (14) des Mehrkanthohlprofils (M) gebildet ist, und wobei die zweite Fläche (131) und die dritte Fläche (151) durch jeweils einen Flächenabschnitt einer Wandungsfläche (W, W') gebildet sind, die zwischen einer Innenfläche (13, 15) und einer Außenfläche (7, 9) erstreckt sind.
